# EUROPÄISCHE PATENTSCHRIFT

(11) **EP 2 884 941 B1**
(45) Veröffentlichungstag und Bekanntmachung des Hinweises auf die Patenterteilung: **07.10.2020**
(21) Anmeldenummer: 13762075.3
(22) Anmeldetag: 14.08.2013
(51) Int. Cl.: A61C 9/00, A61B 5/00, G06T 7/00

(54) **VERFAHREN ZUR REGISTRIERUNG VON EINZELNEN DREIDIMENSIONALEN OPTISCHEN AUFNAHMEN EINES DENTALEN OBJEKTS**
METHOD FOR RECORDING INDIVIDUAL THREE-DIMENSIONAL OPTICAL IMAGES OF A DENTAL OBJECT
PROCÉDÉ POUR ENREGISTRER DES PRISES DE VUES OPTIQUES TRIDIMENSIONNELLES INDIVIDUELLES D'UN OBJET DENTAIRE

(30) Priorität: 14.08.2012 DE 102012214467
(43) Veröffentlichungstag der Anmeldung: 24.06.2015
(73) Patentinhaber: Sirona Dental Systems GmbH, 64625 Bensheim (DE)
(72) Erfinder: ADAMSON, Anders, 64297 Darmstadt (DE)
(74) Vertreter: Özer, Alpdeniz
(86) Internationale Anmeldenummer: PCT/EP2013/066995
(87) Internationale Veröffentlichungsnummer: WO 2014/027026

(56) Entgegenhaltungen:
- US-B1- 6 771 809
- US-B2- 7 698 068

## Beschreibung

### Technisches Gebiet

Die Erfindung betrifft ein Verfahren zur Registrierung von einzelnen dreidimensionalen optischen Aufnahmen zu einer Gesamtaufnahme eines zu vermessenden dentalen Objekts.

### Stand der Technik

US 7,698,068 B2 betrifft ein Verfahren zur Erzeugung von farbigen 3D-Aufnahmen von dentalen Objekten. Aus mehreren Einzelaufnahmen einer Region eines Objekts werden Farbbilder mit Tiefeninformationen, also farbige 3D-Bilder dieser Region erzeugt, wobei aus mehreren chromatischen Bildern ein 3D-Farbbild durch Zusammenfügen erzeugt wird. Auf diese Weise werden mehrere 3D-Farbbilder aufgenommen, die anhand von Überlappungsbereichen zu einem Gesamtbild zusammengefügt werden können.

Probleme des Zusammenfügens, die sich beispielsweise durch das Aufnehmen bewegter Bereiche, wie einer Zunge, ergeben, können dadurch gelöst werden, dass das Zusammenfügen beispielsweise allein anhand der Zahnhartsubstanz vorgenommen wird.

US 6,771,809 B1 offenbart ein Verfahren zum Zusammenfügen einzelner dreidimensionaler Aufnahmen anhand von Überlappungsbereichen zu einer Gesamtaufnahme, wobei die Qualität dieser Überlappungsbereiche überprüft wird. Die Qualitätskontrolle scheint sich dahingehend auszuwirken, dass neue Anfangsparameter zum Auffinden des Überlappungsbereichs bzw. für das Zusammenfügen der Aufnahmen verwendet und das Bestimmen des Überlappungsbereichs bzw. das Zusammenfügen wiederholt wird, bis der Überlappungsbereich eine ausreichende Qualität aufweist. Nach einem Abbruch der wiederholten Versuche des Zusammenfügens wird eine Registrierungsfehlerbehandlungsprozedur ("registration error handling") durchgeführt, die jedoch nicht näher beschrieben ist.

Aus dem Stand der Technik sind weitere Registrierungsverfahren bekannt, bei denen mehrere optische Einzelaufnahmen durchgeführt werden und anschließend eine Registrierung erfolgt. Bei der Registrierung werden übereinstimmende Bereiche, sogenannte Überlappungsbereiche, erkannt und zueinander registriert, so dass aus den einzelnen optischen Aufnahmen eine Gesamtaufnahme gebildet wird.

Beim Zusammenfügen der einzelnen Aufnahmen kann es zu einer fehlerhaften Registrierung kommen. Dies kann beispielsweise durch zu kleine Überlappungsbereiche, durch Aufnahmefehler oder durch störende Objekte im Aufnahmebereich, wie Wange oder Zunge, verursacht werden.

Ein Nachteil solcher Registrierungsverfahren besteht darin, dass eine fehlerhafte Dosierung zu einer verzerrten dreidimensionalen Aufnahme des Objekts führen kann und damit Behandlungsfehler verursachen kann.

Ein weiterer Nachteil dieses Verfahrens besteht darin, dass falls festgestellt wird, dass eine Registrierung fehlerhaft ist, die gesamte Vermessung wiederholt werden muss.

Die Aufgabe der vorliegenden Erfindung besteht daher darin, ein Registrierungsverfahren bereitzustellen, dass eine schnelle und fehlerfreie dreidimensionale Vermessung des dentalen Objekts ermöglicht.

### Darstellung der Erfindung

Die Erfindung wird in Anspruch 1 definiert.

Die Erfindung betrifft ein Verfahren zur Registrierung von einzelnen dreidimensionalen optischen Aufnahmen zu einer Gesamtaufnahme eines zu vermessenden dentalen Objekts, wobei nach jeder einzelnen Aufnahme mittels einer dentalen Kamera unter Verwendung eines Computers automatisch überprüft wird, ob ein Überlappungsbereich zwischen den zusammenzufügenden Aufnahmen bestimmte Registrierungsbedingungen für eine fehlerfreie Registrierung erfüllt. Falls der Überlappungsbereich die Registrierungsbedingungen erfüllt, wird die Registrierung zwischen den zusammenzufügenden Aufnahmen durchgeführt und eine erste Aufnahmesequenz fortgesetzt, wobei die Aufnahmen der ersten Aufnahmesequenz zu einem ersten Cluster zusammengefügt werden. Falls jedoch der Überlappungsbereich einer Aufnahme die Registrierungsbedingungen nicht erfüllt, wird die erste Aufnahmesequenz abgebrochen und automatisch eine weitere zweite Aufnahmesequenz mit dieser Aufnahme begonnen, wobei die Aufnahmen der zweiten Aufnahmesequenz zu einem zweiten Cluster zusammengefügt werden.

Mehrere Cluster werden bei mehreren Aufnahmesequenzen gebildet, wobei die einzelnen Cluster zu der Gesamtaufnahme des Objekts zusammengefügt werden, wobei während der Vermessung automatisch in regelmäßigen Zeitabständen oder auch nach der Vermessung automatisch überprüft wird, ob ein momentaner Cluster mit den vorangehenden Clustern einen Cluster-Überlappungsbereich aufweist und ob dieser Cluster-Überlappungsbereich die Registrierungsbedingungen erfüllt.

Während der Vermessung wird also eine aktuelle Aufnahme zu der vorherigen Aufnahme registriert und zu dem momentanen Cluster hinzugefügt, solange die Registrierungsbedingungen erfüllt sind und es nicht zu einem Abbruch der Aufnahmesequenz kommt. Während dieses Hinzufügens wird das momentane Cluster mit den vorangehenden Clustern, die zuvor vermessen wurden, verglichen. Dabei wird überprüft, ob das momentane Cluster einen Cluster-Überlappungsbereich mit den vorangehenden Clustern aufweist und ob dieser Cluster-Überlappungsbereich die bereits bei der Registrierung der einzelnen Aufnahmen verwendeten Registrierungsbedingungen erfüllt.

Dadurch werden die einzelnen Cluster automatisch zu der Gesamtaufnahme zusammengefügt, sobald ein ausreichender Cluster-Überlappungsbereich festgestellt wird. Die Cluster müssten also nicht manuell zusammengefügt werden, so dass dadurch die Vermessungsdauer verkürzt wird.

Dadurch wird der momentane Cluster mit den vorangehenden Clustern in kurzen Zeitabständen verglichen, um passende Cluster-Überlappungsbereiche zu finden.

Die optischen Aufnahmen werden mittels der dentalen Kamera vermessen, die beispielsweise nach einem Streifenprojektionsverfahren funktionieren kann. Bei dem Streifenprojektionsverfahren können die einzelnen auf das Objekt projizierten Streifen anhand der Intensität, Farbe, Polarisation, Kohärenz, Phase, Kontrast, Ort oder Laufzeit identifiziert werden. Anschließend werden unter Verwendung eines Triangulationsverfahrens die 3D- Koordinaten der einzelnen Messpunkte auf dem Objekt berechnet. Bei der Codierung durch die Farbe kann anhand einer bestimmten Reihenfolge der Farbstreifen jedes der Farbstreifen eindeutig identifiziert werden. Zur Vermessung kann beispielsweise ein Dia bzw. Gitter mit 140 Farbstreifen verwendet werden, die eine Streifenbreite von 130 µm im Messvolumen am Objekt aufweisen. Diese Farbstreifen können beispielsweise acht unterschiedliche Farben aufweisen, wobei die Abfolge einer Gruppe aus drei Farbstreifen über 64 Farbstreifen eindeutig ist.

Während der Vermessung wird die handgehaltene dentale Kamera relativ zum dentalen Objekt, wie einem Unterkiefer oder Oberkiefer, bewegt, wobei in regelmäßigen Zeitabständen die dreidimensionalen optischen Aufnahmen erzeugt werden. Die einzelnen Aufnahmen können beispielsweise mit einer Taktfrequenz zwischen 10 Hz und 20 Hz erzeugt werden. Die Registrierung erfolgt mittels eines Computers, der die aufgenommenen Aufnahmen auswertet. Als Registrierungsverfahren kann beispielsweise das ICP-Registrierungsverfahren (Iterative-Closest-Point-Algorithmus) verwendet werden. Dieser Algorithmus ist ein bekanntes Verfahren zur Registrierung von zweidimensionalen bzw. dreidimensionalen Objekten. Das Ziel dieses Verfahrens ist es zwei unterschiedliche 3D-Modelle eines Objekts annäherungsweise genau aufeinander abzubilden. Dazu werden unterschiedliche Rotationen und Translationen auf korrespondierende Punktepaare der beiden 3D-Modelle angewendet und dabei ein quadratischer Fehler der Abstände zwischen den Punktepaaren minimiert. Dafür werden im ersten Schritt die nächsten Nachbarn eines bestimmten Punktes ermittelt. Im nächsten Schritt wird die Transformation zur Registrierung berechnet. Daraufhin wird die errechnete Transformation auf die zu registrierenden Punktepaare angewendet. Diese iterative Annäherung wird solange ausgeführt, bis die beiden 3D-Modelle im Überlagerungsbereich übereinstimmen.

Alternativ oder ergänzend dazu kann die Registrierung auch aufgrund der Farbe des aufgenommenen Objekts, der Oberflächenkrümmung des aufgenommenen Objekts oder aufgrund von geometrischen Übereinstimmungen erfolgen. Bei der Registrierung aufgrund geometrischer Übereinstimmungen wird ein Mustererkennungsalgorithmus verwendet, bei dem die zweite Aufnahme nach einem bestimmten geometrischen Muster, wie nach einer Okklusionsfläche eines bestimmten Zahns, aus der ersten Aufnahme durchsucht wird.

Falls die Registrierungsbedingungen für den Überlappungsbereich nicht erfüllt werden, wird ein neues begonnen. Während der Vermessung des dentalen Objekts wird also solange nach jedem Abbruch ein neues Cluster begonnen, bis das gesamte Objekt vermessen wurde. Die einzelnen Cluster können dann zu der Gesamtaufnahme des Objekts zusammengefügt werden. Die Registrierungsbedingungen werden beispielsweise in Fällen nicht erfüllt, wenn die Dentalkamera in Relation zum Objekt zu schnell bewegt wird, und dadurch die Größe des Überlappungsbereichs unzureichend ist. Ein weiterer Grund könnte sein, dass ein Autofokus der dentalen Kamera unscharf eingestellt ist und dadurch das Objekt undeutlich abgebildet wird, so dass die Aufnahmequalität der Aufnahme nicht ausreichend ist. Ein weiterer Grund könnte sein, dass bewegliche Objekte, wie Zunge des Patienten oder Finger des behandelnden Zahnarztes, während der Vermessung erfasst werden. Dies führt dazu, dass die Überlappungsbereiche der Aufnahmen nicht übereinstimmen. Die genannten Gründe können also zu einem Abbruch der Aufnahmesequenz führen.

Ein Vorteil dieses Verfahrens besteht darin, dass die Registrierungsbedingungen nach jeder Aufnahme überprüft werden, so dass es dadurch nicht zu einer fehlerhaften Registrierung kommen kann.

Ein weiterer Vorteil dieses Verfahrens besteht darin, dass die bereits erzeugten Aufnahmen nach einem Abbruch der Aufnahmesequenz nicht verworfen werden, sondern zu einem Cluster zusammengefügt werden. Anschließend können die einzelnen Cluster automatisch verglichen werden und zu der Gesamtaufnahme des Objekts zusammengefügt werden, so dass einzelne Bereiche des dentalen Objekts nicht mehrfach vermessen werden müssen. Dadurch wird die Vermessungsdauer verkürzt.

Vorteilhafterweise können die Registrierungsbedingungen eine ausreichende Größe des Überlappungsbereichs, eine ausreichende Welligkeit der Objektoberfläche im Überlappungsbereich, eine ausreichende Rauheit der Oberfläche im Überlappungsbereich, eine ausreichende Anzahl von charakteristischen Geometrien im Überlappungsbereich und/oder ausreichende Aufnahmequalität der Aufnahme im Überlappungsbereich sein.

Dadurch wird eine fehlerhafte Registrierung zwischen den zusammenzufügenden Aufnahmen verhindert. Durch die ausreichende Helligkeit und Rauheit der Oberfläche des Überlappungsbereich wird im Gegensatz zu einer ebenen Fläche eine zuverlässige Registrierung ermöglicht. Die ausreichende Anzahl und Anordnung der charakteristischen Geometrien, wie beispielsweise von Fissuren oder Zahnhöckern, ermöglicht ebenfalls eine zuverlässige Registrierung. Bei einer ausreichenden Aufnahmequalität wird das dentale Objekt scharf und kontrastreich abgebildet. Ein Grund für geringen Kontrast könnte beispielsweise eine unzureichende Beleuchtung des Objekts sein. Ein Grund für die unscharfe Aufnahme könnte beispielsweise ein fehlerhaft eingestellter Autofokus sein.

Vorteilhafterweise kann die ausreichende Größe des Überlappungsbereichs mindestens ein Viertel einer Aufnahmeoberfläche der jeweiligen Aufnahme betragen.

Durch einen solchen Überlappungsbereich wird eine fehlerfreie Registrierung gewährleistet.

Vorteilhafterweise kann die Überprüfung des momentanen Clusters in regelmäßigen Zeitabständen jeweils nach jeden 10 bis 40 Aufnahmen erfolgen.

Dadurch wird der wachsende momentane Cluster in kurzen Zeitabständen nach jeden 10 bis 40 Aufnahmen mit den vorhergehenden Clustern verglichen.

Vorteilhafterweise kann die Überprüfung des momentanen Clusters erfolgen, sobald eine seit einer vorangehenden Überprüfung zum momentanen Cluster hinzugefügte Fläche eine Fläche von mindestens 0,25 cm² überschreitet.

Dadurch erfolgt die Überprüfung erst wenn die hinzugefügte Fläche mindestens 0,25 cm² überschreitet. Dies hat den Vorteil, dass die Überprüfung erst erfolgt, wenn die dentale Kamera relativ zum Objekt bewegt wird.

Vorteilhafterweise können der momentane Cluster und die vorangehenden Cluster mittels einer Anzeigevorrichtung grafisch gleichzeitig angezeigt werden, wobei das Zusammenfügen der einzelnen Cluster zu der Gesamtaufnahme des Objekts während der Vermessung dargestellt wird.

Dadurch werden die bereits registrierten vorangehenden Cluster und der momentane Cluster mittels der Anzeigevorrichtung, wie mittels eines Monitors, angezeigt, um dem Benutzer einen besseren Überblick über die Aufnahmesituation oder den Aufnahmeprozess zu geben. Dies ermöglicht dem Benutzer bereits während der Vermessung zu sehen, welche Bereiche des dentalen Objekts bereits vermessen wurden und welche nicht. Der Benutzer könnte also gezielt Bereiche vermessen, die zwischen den bereits registrierten Clustern liegen, und die Cluster miteinander zu registrieren.

Durch das Anzeigen der hinzugefügten Fläche des momentanen Clusters sieht der Benutzer in welche Richtung der Cluster wächst. Das Anzeigen der Aufnahmerichtung der einzelnen Cluster erleichtert dem Benutzer zusätzlich die Orientierung, um festzustellen welche Bereiche des dentalen Objekts noch nicht vermessen wurden. Das Anzeigen des Abbruchs ermöglicht dem Benutzer festzustellen, an welcher Stelle der jeweilige Cluster abgebrochen wurde. Der Benutzer könnte also gezielt den momentanen Cluster in Richtung einer Abbruchstelle der vorherigen Cluster bewegen, um einen ausreichenden Cluster-Überlappungsbereich zu erzeugen.

Vorteilhafterweise kann die hinzugefügte Fläche des momentanen Clusters, eine Aufnahmerichtung der einzelnen Cluster und/oder die Position eines Abbruchs der bereits registrierten Cluster mittels der Anzeigevorrichtung graphisch dargestellt werden.

Das Anzeigen der Aufnahmedichtung und der Abbruchspositionen ermöglicht dem Benutzer einen besseren Überblick über den Aufnahmeprozess.

Vorteilhafterweise kann die Registrierung die einzelnen Cluster während der Vermessung des Objekts ohne Absetzen der Kamera ablaufen.

Dadurch erfolgt die Vermessung des Objekts kontinuierlich, wobei nach jedem Abbruch ein neuer Cluster gebildet wird.

Vorteilhafterweise kann die Registrierung der Aufnahmen und/oder der Cluster unter Verwendung von semantischen Strukturen erfolgen, nämlich anhand eines ermittelten Verlaufs eines zu vermessenden Kieferbogens, einer Okklusionsrichtung und/oder anhand von Zahnzentren.

Die semantischen Strukturen können dabei unter Verwendung einer Datenbank erkannt werden, die mehrere Datensätze von Kieferbögen, Zähnen und Okklusionsflächen verschiedener Patienten umfasst. Aufgrund dieser zusätzlichen Informationen wird die Lagebeziehung zwischen den zusammenzufügenden Aufnahmen überprüft und dadurch die Registrierung verbessert. Die zusätzlichen Informationen aufgrund der semantischen Strukturen vereinfachen auch das mathematische Problem der Registrierung, so dass dadurch die Rechenzeit verkürzt wird.

Vorteilhafterweise kann die Okklusionsrichtung mittels des Computers automatisch unter Verwendung eines Analyseverfahrens der aufgenommenen Aufnahmen ermittelt werden, wobei Oberflächennormalen von Zahnoberflächen der vermessenen Zähne des Objekts erzeugt werden und ein Mittelwert der Oberflächennormalen einer Okklusionsfläche die Okklusionsrichtung eines bestimmten Zahns bildet.

Dadurch wird die Okklusionsrichtung automatisch unter Verwendung des Analyseverfahrens ermittelt.

Vorteilhafterweise kann die Registrierung der Aufnahmen und/oder der Cluster unter Verwendung einer Bildfeldregistrierung erfolgen, wobei die zusammenzufügenden Aufnahmen oder Cluster in mehrere Teilbereiche mit einer definierten Größe unterteilt werden und anschließend die Teilbereiche nach einer festgelegten Reihenfolge miteinander verglichen werden, um den Überlappungsbereich beziehungsweise den Cluster-Überlappungsbereich zu finden, der die Registrierungsbedingungen erfüllt.

Dadurch werden lediglich die kleinen Teilbereiche miteinander verglichen, so dass dadurch die Rechenzeit für die Registrierung verkürzt wird.

### Kurzbeschreibung der Zeichnungen

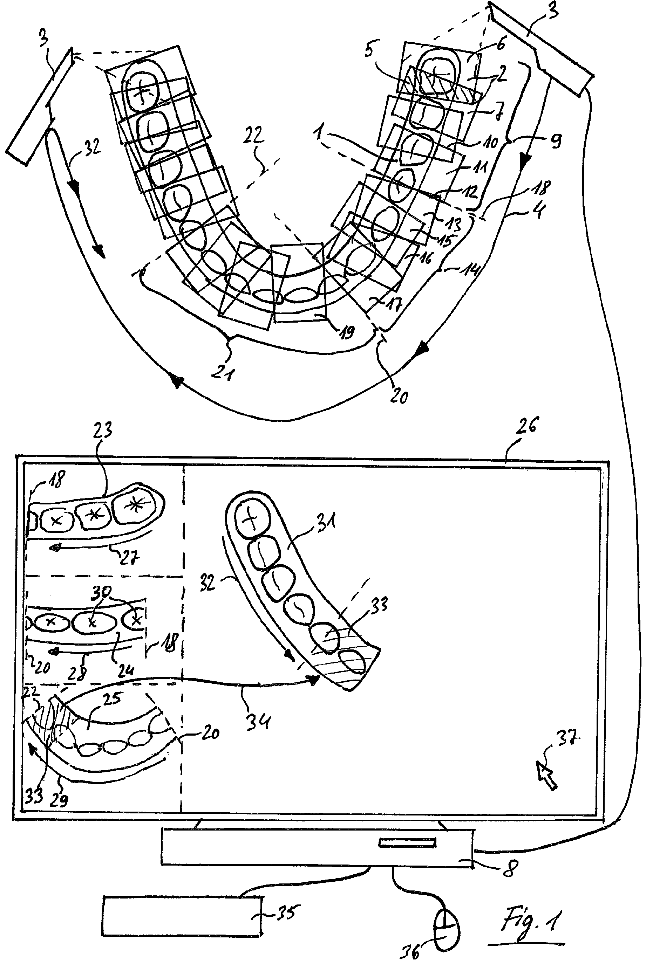

Die Erfindung wird anhand der Zeichnungen erläutert. Es zeigt, die
- Fig. 1: eine Skizze zur Verdeutlichung des vorliegenden Verfahrens zur Registrierung von einzelnen Aufnahmen.

### Ausführungsbeispiel

Die Fig. 1 zeigt eine Skizze zur Verdeutlichung des vorliegenden Verfahrens zur Registrierung von einzelnen Aufnahmen eines zu vermessenden dentalen Objekts 1, wie eines Unterkiefers. Die dreidimensionalen optischen Aufnahmen 2, die in Form von Rechtecken dargestellt sind, werden mittels einer dentalen Kamera 3 vermessen, die während der Vermessung relativ zum Objekt 1 entlang eines Vermessungswegs 4 bewegt wird. Die Dentalkamera 3 kann beispielsweise eine handgehaltene Kamera sein, die das Objekt 1 unter Verwendung eines Streifenprojektionsverfahrens vermisst. Ein erster Überlappungsbereich 5 zwischen einer ersten Aufnahme 6 und einer zweiten Aufnahme 7, der gestrichelt dargestellt ist, wird unter Verwendung eines Computers 8 hinsichtlich Registrierungsbedingungen überprüft. Dabei wird überprüft, ob der Überlappungsbereich eine ausreichende Größe, eine ausreichende Welligkeit, eine ausreichende Rauheit, eine ausreichende Aufnahmequalität und/oder eine ausreichende Anzahl und Anordnung von charakteristischen Geometrien aufweist. Beispielsweise kann überprüft werden, ob der Überlappungsbereich mindestens ein Viertel der Oberfläche der ersten Aufnahme 6 beträgt. Der erste Überlappungsbereich 5 erfüllt die Registrierungsbedingungen, so dass eine erste Aufnahmesequenz 9 bestehend aus der ersten Aufnahme 6, der zweiten Aufnahme 7 einer dritten Aufnahme 10 und einer vierten Aufnahme 11, fortgesetzt wird. Ein zweiter Überlappungsbereich 12 zwischen der vierten Aufnahme 11 und einer fünften Aufnahme 13 erfüllt jedoch nicht die Registrierungsbedingungen, da der zweite Überlappungsbereich zu klein ist. Daraufhin wird die erste Aufnahmesequenz abgebrochen und automatisch eine weitere zweite Aufnahmesequenz 14 bestehend aus der fünften Aufnahme 13, der sechsten Aufnahme 15 der siebenten Aufnahme 16 und der achten Aufnahme 17, begonnen. Der erste Abbruch 18 der ersten Aufnahmesequenz 9 ist durch eine gestrichelte Linie dargestellt. Zwischen der achten Aufnahme 17 und einer neunten Aufnahme 19 folgt ein zweiter Abbruch 20 der zweiten Aufnahmesequenz 14 anschließend wird eine dritte Aufnahmesequenz 21 bis zu einem dritten Abbruch 22 vermessen. Mittels eines Computers 8 werden die Aufnahmen der ersten Aufnahmesequenz 9 zu einem ersten Cluster 23, die Aufnahmen der zweiten Aufnahmesequenz 14 zu einem zweiten Cluster 24 und die Aufnahmen der dritten Aufnahmesequenz 21 zu einem dritten Cluster 25 zusammengefügt. Mittels einer Anzeigevorrichtung 26, wie mittels eines Monitors, werden die Cluster 23, 24 und 25 angezeigt. Zur leichteren Orientierung wird eine erste Aufnahmerichtung 27, eine zweite Aufnahmerichtung 28 des zweiten Clusters 24 und eine dritte Aufnahmerichtung 29 des dritten Clusters 25 durch Pfeile dargestellt. Ergänzend können auch semantische Strukturen, wie Zahnzentren 30 angezeigt werden. Die Positionen der Abbrüche 18 können auch angezeigt werden. Daraufhin wird ein vierter momentaner Cluster 31 in eine vierte Aufnahmerichtung 32 vermessen. Dabei wird in regelmäßigen Zeitabständen automatisch überprüft, ob der momentane Cluster 31 mit den vorher registrierten Clustern 23, 24 und 25 einen Cluster-Überlappungsbereich 33 aufweist, der gestrichelt dargestellt ist und ob dieser Cluster-Überlappungsbereich 33 die Registrierungsbedingungen erfüllt. Diese Überprüfung kann beispielsweise in regelmäßigen Zeitabständen jeweils nach jeden 10 bis 40 Aufnahmen erfolgen. Anschließend wird eine Registrierung zwischen dem dritten Cluster 25 und dem vierten Cluster 31 durchgeführt, wobei das dritte Cluster 25 mit dem vierten Cluster 31 zusammengefügt wird, wie durch den Pfeil 24 dargestellt ist. Auf diese Weise können weitere Cluster an den Abbruchstellen 18 und 20 vermessen werden und alle Cluster 23, 24, 25 und 31 zu einer Gesamtaufnahme des Objekts 1 zusammengefügt werden.

Alternativ dazu können die einzelnen Cluster 23, 24, 25 und 31 auch manuell durch den Benutzer unter Verwendung von Eingabemitteln, wie einer Tastatur 35 und einer Maus 36, über einen Cursor 37 zu der Gesamtaufnahme zusammengefügt werden.

### Bezugszeichen

- 1: Objekt
- 2: Aufnahme
- 3: Kamera
- 4: Vermessungsweg
- 5: erster Überlappungsbereich
- 6: erste Aufnahme
- 7: zweite Aufnahme
- 8: Computer
- 9: erste Aufnahmesequenz
- 10: dritte Aufnahme
- 11: vierte Aufnahme
- 12: zweiter Überlappungsbereich
- 13: fünfte Aufnahme
- 14: zweite Aufnahmesequenz
- 15: sechste Aufnahme
- 16: siebte Aufnahme
- 17: achte Aufnahme
- 18: erster Abbruch
- 19: neunte Aufnahme
- 20: zweiter Abbruch
- 21: dritte Aufnahmesequenz
- 22: dritter Abbruch
- 23: erster Cluster
- 24: zweiter Cluster
- 25: dritter Cluster
- 26: Anzeigevorrichtung
- 27: erste Aufnahmerichtung
- 28: zweite Aufnahmerichtung
- 29: dritte Aufnahmerichtung
- 30: Zahnzentren
- 31: vierter Cluster
- 32: vierte Aufnahmerichtung
- 33: Cluster-Überlappungsbereich
- 34: Pfeil
- 35: Tastatur
- 36: Maus
- 37: Cursor

## Patentansprüche

1. Verfahren zur Registrierung von einzelnen dreidimensionalen optischen Aufnahmen (2) zu einer Gesamtaufnahme eines zu vermessenden dentalen Objekts (1), wobei nach jeder einzelnen Aufnahme (2, 6, 7, 10, 11, 13, 15, 16, 17, 19) mittels einer dentalen Kamera (3) unter Verwendung eines Computers (8) automatisch überprüft wird, ob ein Überlappungsbereich (5, 12) zwischen den zusammenzufügenden Aufnahmen (6, 7, 11, 13) bestimmte Registrierungsbedingungen für eine fehlerfreie Registrierung erfüllt, wobei falls der Überlappungsbereich (5) die Registrierungsbedingungen erfüllt, die Registrierung zwischen den zusammenzufügenden Aufnahmen (6, 7) durchgeführt wird und eine erste Aufnahmesequenz (9) fortgesetzt wird, wobei die Aufnahmen (6, 7, 10, 11) der ersten Aufnahmesequenz (9) zu einem ersten Cluster (23) zusammengefügt werden, oder falls der Überlappungsbereich (12) einer Aufnahme (13) die Registrierungsbedingungen nicht erfüllt, die erste Aufnahmesequenz (9) abgebrochen wird und automatisch eine weitere zweite Aufnahmesequenz (14) mit dieser Aufnahme (13) begonnen wird, wobei die Aufnahmen (13, 15, 16, 17) der zweiten Aufnahmesequenz (14) zu einem zweiten Cluster (24) zusammengefügt werden, wobei mehrere Cluster (23, 24, 25, 31) bei mehreren Aufnahmesequenzen (9, 14, 21) gebildet werden, wobei die einzelnen Cluster (23, 24, 25, 31) zu der Gesamtaufnahme des Objekts (1) zusammengefügt werden, **dadurch gekennzeichnet, dass** während der Vermessung automatisch in regelmäßigen Zeitabständen oder nach der Vermessung automatisch überprüft wird, ob ein momentaner Cluster (31) mit den vorangehenden Clustern (23, 24, 25) einen Cluster-Überlappungsbereich (33) aufweist und ob dieser Cluster-Überlappungsbereich (33) die Registrierungsbedingungen erfüllt.

2. Verfahren nach Anspruch 1, **dadurch gekennzeichnet, dass** die Registrierungsbedingungen eine ausreichende Größe des Überlappungsbereichs (5, 12), eine ausreichende Welligkeit der Objektoberfläche im Überlappungsbereich (5, 12), eine ausreichende Rauheit der Oberfläche im Überlappungsbereich (5, 12), eine ausreichende Anzahl von charakteristischen Geometrien im Überlappungsbereich (5, 12) und/oder ausreichende Aufnahmequalität der Aufnahme im Überlappungsbereich (5, 12) sind.

3. Verfahren nach Anspruch 2, **dadurch gekennzeichnet, dass** die ausreichende Größe des Überlappungsbereichs (5) mindestens ein Viertel einer Aufnahmeoberfläche der jeweiligen Aufnahme (7) beträgt.

4. Verfahren nach Anspruch 1, **dadurch gekennzeichnet, dass** die Überprüfung des momentanen Clusters (31) in regelmäßigen Zeitabständen jeweils nach jeden 10 bis 40 Aufnahmen (2) erfolgt.

5. Verfahren nach Anspruch 1, **dadurch gekennzeichnet, dass** die Überprüfung des momentanen Clusters (31) erfolgt, sobald eine seit einer vorangehenden Überprüfung zum momentanen Cluster (31) hinzugefügte Fläche eine Fläche von mindestens 0,25 cm² überschreitet.

6. Verfahren nach einem der Ansprüche 1 bis 5, **dadurch gekennzeichnet, dass** der momentane Cluster (31) und die vorangehenden Cluster (23, 24, 25) mittels einer Anzeigevorrichtung (26) grafisch gleichzeitig angezeigt werden, wobei das Zusammenfügen (34) der einzelnen Cluster (23, 24, 25, 31) zu der Gesamtaufnahme des Objekts (1) während der Vermessung dargestellt wird.

7. Verfahren nach Anspruch 6, **dadurch gekennzeichnet, dass** die hinzugefügte Fläche des momentanen Clusters (31), eine Aufnahmerichtung (27, 28, 29, 32) der einzelnen Cluster (23, 24, 25, 31) und/oder die Position eines Abbruchs (18, 20, 22) der bereits registrierten Cluster mittels der Anzeigevorrichtung (26) graphisch dargestellt werden.

8. Verfahren nach einem der Ansprüche 1 bis 7, **dadurch gekennzeichnet, dass** die Registrierung der einzelnen Cluster (23, 24, 25, 31) während der Vermessung des Objekts (1) ohne Absetzen der Kamera (3) abläuft.

9. Verfahren nach einem der Ansprüche 1 bis 8, **dadurch gekennzeichnet, dass** die Registrierung der Aufnahmen (2) und/oder der Cluster (23, 24, 25, 31) unter Verwendung von semantischen Strukturen erfolgt, nämlich anhand eines ermittelten Verlaufs eines zu vermessenden Kieferbogens, einer Okklusionsrichtung und/oder anhand von Zahnzentren (30).

10. Verfahren nach Anspruch 9, **dadurch gekennzeichnet, dass** die Okklusionsrichtung mittels des Computers (8) automatisch unter Verwendung eines Analyseverfahrens der aufgenommenen Aufnahmen (2) ermittelt wird, wobei Oberflächennormalen von Zahnoberflächen der vermessenen Zähne des Objekts (1) erzeugt werden und ein Mittelwert der Oberflächennormalen einer Okklusionsfläche die Okklusionsrichtung eines bestimmten Zahns bildet.

11. Verfahren nach einem der Ansprüche 1 bis 10, **dadurch gekennzeichnet, dass** die Registrierung der Aufnahmen (2) und/oder der Cluster (23, 24, 25, 31) unter Verwendung einer Bildfeldregistrierung erfolgt, wobei die zusammenzufügenden Aufnahmen (2) oder Cluster (23, 24, 25, 31) in mehrere Teilbereiche mit einer definierten Größe unterteilt werden und anschließend die Teilbereiche nach einer festgelegten Reihenfolge miteinander verglichen werden, um den Überlappungsbereich (5, 12) beziehungsweise den Cluster-Überlappungsbereich (33) zu finden, der die Registrierungsbedingungen erfüllt.

## Claims

1. A method for recording individual three-dimensional optical images (2) for an overall image of a dental object (1) to be measured, wherein after each individual image (2, 6, 7, 10, 11, 13, 15, 16, 17, 19) by means of a dental camera (3), a computer (8) is used to automatically check whether an overlap region (5, 12) between the images (6, 7, 11, 13) to be combined fulfils certain recording conditions for error-free recording, wherein if the overlap region (5) fulfils the recording conditions, the recording is carried out between the images (6, 7) to be combined and a first image sequence (9) is continued, wherein the images (6, 7, 10, 11) of the first image sequence (9) are combined into a first cluster (23), or if the overlap region (12) of an image (13) does not fulfil the recording conditions, the first image sequence (9) is terminated and another, second image sequence (14) is automatically started with said image (13), wherein the images (13, 15, 16, 17) of the second image sequence (14) are combined into a second cluster (24), wherein a plurality of clusters (23, 24, 25, 31) is formed in the case of a plurality of image sequences (9, 14, 21), wherein the individual clusters (23, 24, 25, 31) are combined into the overall image of the object (1), **characterised in that** it is automatically checked at regular intervals during the measurement or after the measurement as to whether a current cluster (31) shows a cluster overlap region (33) with the preceding clusters (23, 24, 25) and whether said cluster overlap region (33) fulfils the recording conditions.

2. The method according to claim 1, **characterised in that** the recording conditions are a sufficient size of the overlap region (5, 12), a sufficient waviness of the object surface in the overlap region (5, 12), a sufficient roughness of the surface in the overlap region (5, 12), a sufficient number of characteristic geometries in the overlap region (5, 12) and/or sufficient image quality of the recorded image in the overlap region (5, 12).

3. The method according to claim 2, **characterised in that** the sufficient size of the overlap region (5) is at least a quarter of an image surface of the image (7).

4. The method according to claim 1, **characterised in that** the current cluster (31) is checked at regular intervals in each case after every 10 to 40 images (2).

5. The method according to claim 1, **characterised in that** the current cluster (31) is checked as soon as an area added to the current cluster (31) since a previous check exceeds an area of at least 0.25 cm².

6. The method according to any one of claims 1 to 5, **characterised in that** the current cluster (31) and the preceding clusters (23, 24, 25) are graphically displayed simultaneously by means of a display device (26), wherein the merging (34) of the individual clusters (23, 24, 25, 31) for the overall image of the object (1) is displayed during measurement.

7. The method according to claim 6, **characterised in that** the added area of the current cluster (31), an image direction (27, 28, 29, 32) of the individual clusters (23, 24, 25, 31) and/or the position of an abort (18, 20, 22) of the already recorded clusters are graphically displayed by means of the display device (26).

8. The method according to any of claims 1 to 7, **characterised in that** the recording of the individual clusters (23, 24, 25, 31) takes place during the measurement of the object (1) without putting the camera (3) down.

9. The method according to any of claims 1 to 8, **characterised in that** the recording of the images (2) and/or the clusters (23, 24, 25, 31) is carried out using semantic structures, namely on the basis of a determined profile of a jaw arch to be measured, an occlusion direction and/or on the basis of tooth centres (30).

10. The method according to claim 9, **characterised in that** the occlusion direction is automatically determined by means of the computer (8) using a method of analysis of the recorded images (2), wherein surface normals are generated from tooth surfaces of the measured teeth of the object (1) and an average value of the surface normals of an occlusion surface forms the occlusion direction of a particular tooth.

11. The method according to any of claims 1 to 10, **characterised in that** the recording of the images (2) and/or the clusters (23, 24, 25, 31) is carried out using image field recording, wherein the images (2) or clusters (23, 24, 25, 31) to be combined are divided into a plurality of sub-regions of a defined size and the sub-regions are then compared with one another in a defined order to find the overlap region (5, 12) or the cluster overlap region (33) that fulfils the recording conditions.

## Revendications

1. Procédé pour enregistrer des prises de vues optiques tridimensionnelles individuelles (2) pour une prise de vue globale d'un objet dentaire (1) à mesurer, après chaque prise de vue individuelle (2, 6, 7, 10, 11, 13, 15, 16, 17, 19) au moyen d'une caméra dentaire (3), la prise de vue étant automatiquement vérifiée en utilisant un ordinateur (8) concernant le fait qu'une zone de chevauchement (5, 12) entre les prises de vues (6, 7, 11, 13) à assembler remplit certaines conditions d'enregistrement pour un enregistrement sans erreur, et si la zone de chevauchement (5) remplit les conditions d'enregistrement, l'enregistrement s'effectuant entre les prises de vues (6, 7) à assembler et une première séquence de prises de vues (9) se poursuivant, les prises de vues (6, 7, 10, 11) de la première séquence de prises de vues (9) étant assemblées en un premier groupe (23), ou si la zone de chevauchement (12) d'une prise de vue (13) ne remplit pas les conditions d'enregistrement, la première séquence de prises de vues (9) étant terminée et automatiquement une autre deuxième séquence de prises de vues (14) étant lancée avec cette prise de vue (13), les prises de vues (13, 15, 16, 17) de la deuxième séquence de prises de vues (14) étant assemblées en un deuxième groupe (24), plusieurs groupes (23, 24, 25, 31) étant assemblés pour une pluralité de séquences de prises de vues (9, 14, 21), les groupes individuels (23, 24, 25, 31) étant assemblés pour former la prise de vue globale de l'objet (1), **caractérisé en ce que** pendant la mesure, on vérifie automatiquement à intervalles réguliers ou après la mesure, si un groupe actuel (31) présente une zone de chevauchement de groupe (33) avec les groupes précédents (23, 24, 25) et si cette zone de chevauchement de groupe (33) remplit les conditions d'enregistrement.

2. Procédé selon la revendication 1, **caractérisé en ce que** les conditions d'enregistrement sont une taille suffisante de la zone de chevauchement (5, 12), une ondulation suffisante de la surface de l'objet dans la zone de chevauchement (5, 12), une rugosité suffisante de la surface dans la zone de chevauchement (5, 12), un nombre suffisant de géométries caractéristiques dans la zone de chevauchement (5, 12) et/ou une qualité de prise de vue suffisante dans la zone de chevauchement (5, 12).

3. Procédé selon la revendication 2, **caractérisé en ce que** la taille suffisante de la zone de chevauchement (5) représente au moins un quart d'une surface de prise de vue de la prise de vue respective (7).

4. Procédé selon la revendication 1, **caractérisé en ce que** le groupe actuel (31) est vérifié à intervalles réguliers toutes les 10 à 40 prises de vues (2).

5. Procédé selon la revendication 1, **caractérisé en ce que** le groupe actuel (31) est vérifié dès qu'une surface ajoutée au groupe actuel (31) d'une vérification précédente dépasse une superficie d'au moins 0,25 cm².

6. Procédé selon l'une quelconque des revendications 1 à 5, **caractérisé en ce que** le groupe actuel (31) et les groupes précédents (23, 24, 25) sont affichés graphiquement simultanément au moyen d'un dispositif d'affichage (26), l'assemblage (34) des groupes individuels (23, 24, 25, 31) en la prise de vue globale de l'objet (1) s'affichant pendant la mesure.

7. Procédé selon la revendication 6, **caractérisé en ce que** la surface ajoutée du groupe actuel (31), une direction de prise de vue (27, 28, 29, 32) des groupes individuels (23, 24, 25, 31) et/ou la position d'une interruption (18, 20, 22) des groupes déjà enregistrés sont affichées graphiquement au moyen du dispositif d'affichage (26).

8. Procédé selon l'une quelconque des revendications 1 à 7, **caractérisé en ce que** l'enregistrement des groupes individuels (23, 24, 25, 31) a lieu lors de la mesure de l'objet (1) sans poser la caméra (3).

9. Procédé selon l'une quelconque des revendications 1 à 8, **caractérisé en ce que** l'enregistrement des prises de vues (2) et/ou des groupes (23, 24, 25, 31) est réalisé en utilisant des structures sémantiques, à savoir sur la base d'un parcours déterminé d'un arc maxillaire à mesurer, d'une direction d'occlusion et/ou sur la base des centres dentaires (30).

10. Procédé selon la revendication 9, **caractérisé en ce que** la direction d'occlusion est déterminée automatiquement au moyen de l'ordinateur (8) en utilisant une méthode d'analyse des prises de vues enregistrées (2), les normales de surface étant générées à partir des surfaces dentaires des dents mesurées de l'objet (1) et une valeur moyenne des normales de surface d'une surface occlusale forme la direction d'occlusion d'une dent particulière.

11. Procédé selon l'une quelconque des revendications 1 à 10, **caractérisé en ce que** l'enregistrement des prises de vues (2) et/ou des groupes (23, 24, 25, 31) est effectué en utilisant un enregistrement de champ d'image, les prises de vues (2) ou groupes (23, 24, 25, 31) assemblés étant divisés en plusieurs zones partielles ayant une taille définie, puis les zones partielles étant comparées les unes aux autres dans un ordre défini afin de trouver la zone de chevauchement (5, 12) ou la zone de chevauchement de groupes (33) qui remplit les conditions d'enregistrement.
